(19) **Europäisches Patentamt**
**European Patent Office**
**Office européen des brevets**

(11)  **EP 3 484 364 B1**

(12)  **FASCICULE DE BREVET EUROPEEN**

(45) Date de publication et mention de la délivrance du brevet:
**09.03.2022 Bulletin 2022/10**

(21) Numéro de dépôt: **17748524.0**

(22) Date de dépôt: **10.07.2017**

(51) Classification Internationale des Brevets (IPC):
**A61B 5/00** *(2006.01)*    **B25J 9/16** *(2006.01)*
**A61B 5/11** *(2006.01)*

(52) Classification Coopérative des Brevets (CPC):
**A61B 5/1114; A61B 5/744; B25J 9/1628;**
G05B 2219/40444

(86) Numéro de dépôt international:
**PCT/FR2017/051876**

(87) Numéro de publication internationale:
**WO 2018/011496 (18.01.2018 Gazette 2018/03)**

(54) **PROCÉDÉ ET SYSTÈME DE COMMANDE HIÉRARCHIQUE EN TEMPS RÉEL D'UNE STRUCTURE ARTICULÉE EXPLOITANT UN NOMBRE LIMITÉ DE CAPTEURS DE MOUVEMENT**

VERFAHREN UND SYSTEM ZUR HIERARCHISCHEN STEUERUNG IN ECHTZEIT EINER GELENKSTRUKTUR UNTER VERWENDUNG EINER BEGRENZTEN ANZAHL VON BEWEGUNGSSENSOREN

METHOD AND SYSTEM FOR THE HIERARCHICAL CONTROL IN REAL TIME OF AN ARTICULATED STRUCTURE UTILISING A LIMITED NUMBER OF MOVEMENT SENSORS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.07.2016 FR 1656739**

(43) Date de publication de la demande:
**22.05.2019 Bulletin 2019/21**

(73) Titulaire: **Commissariat à l'Énergie Atomique et aux Énergies Alternatives 75015 Paris (FR)**

(72) Inventeurs:
- **WEISTROFFER, Vincent** 75005 Paris (FR)
- **ANDRIOT, Claude** 75015 Paris (FR)
- **MICAELLI, Alain** 92260 Fontenay-Aux-Roses (FR)

(74) Mandataire: **Brevalex 95, rue d'Amsterdam 75378 Paris Cedex 8 (FR)**

(56) Documents cités:
WO-A1-2013/131990    FR-A1- 3 000 376
US-A1- 2015 192 413

**Description**

**DOMAINE TECHNIQUE**

Le domaine de l'invention est celui de la capture du mouvement d'un objet, et du transfert de ce mouvement à une structure massique (poly-)articulée modélisant l'objet.

L'invention trouve application à la capture du mouvement d'un corps humain via des capteurs disposés sur une personne, et à la représentation de ce mouvement via l'animation d'un avatar dans une simulation numérique.

**ÉTAT DE LA TECHNIQUE ANTÉRIEURE**

On cherche dans un certain nombre d'applications à représenter les mouvements d'une personne dans une simulation numérique. Dans la majorité des cas, la personne porte un ensemble de capteurs dont les données sont utilisées pour animer son avatar numérique dans la simulation.

Afin de rendre l'animation robuste et réaliste, il est nécessaire que la personne porte un grand nombre de capteurs. On a ainsi généralement recours à un capteur par partie du corps, soit un ensemble d'au moins une vingtaine de capteurs.

L'installation des capteurs est toutefois fastidieuse, coûteuse en temps et intrusive sur la personne. L'utilisation d'un nombre de capteurs réduit permettrait de rendre les dispositifs de capture de mouvement moins contraignants pour les utilisateurs.

Malheureusement, la réduction du nombre de capteurs n'est pas un procédé immédiat. En effet, la disparition de certains capteurs engendre la disparition de contraintes sur le mannequin numérique simulant l'utilisateur, et donc l'apparition de degrés de liberté supplémentaires.

Or avec les techniques de cinématique inverse classiquement utilisées dans le domaine de l'animation, le manque de données ne peut être compensé et les degrés de liberté supplémentaires peuvent entraîner des mouvements non fidèles et non réalistes du mannequin numérique. Dans la majeure partie des cas, ces mouvements sont liés à des problèmes de redondance (pour une position donnée, plusieurs configurations du corps peuvent être possibles, notamment pour les bras ou les jambes) ou de gestion d'équilibre (colonne vertébrale).

Pour répondre à cette problématique, il a été proposé, par exemple par la demande de brevet US 2015/0192413 A1, une approche se reposant sur une base de données (« data-driven »). Selon cette approche, le mouvement détecté au moyen d'un ensemble réduit de cinq capteurs de mouvement est comparé à des mouvements préenregistrés dans une base de données pour identifier un mouvement approchant qui est alors joué par le mannequin numérique.

Une telle approche n'est pas totalement satisfaisante. Elle nécessite en effet la constitution préalable de la base de données. Cette base de données dépend en outre du domaine d'application et n'est donc pas générique. Enfin, des interpolations sont nécessaires pour gérer les mouvements non représentés dans la base de données, ce qui induit un coût de calcul et un rendu approximatif.

On connait par ailleurs de la demande internationale WO 2013/131990 A1 un procédé d'identification de paramètres géométriques de capteurs chacun disposés sur un segment d'un corps humain. Ce procédé comprend l'évaluation de valeurs de paramètres caractérisant l'orientation et la position de chaque capteur dans le repère de chaque segment et de la longueur de chaque segment, dans un repère dudit segment, en utilisant des configurations et des mouvements prédéterminés du corps. Ce procédé permet de procéder à la calibration des capteurs.

Le document FR 3 000 376 A1 décrit quant à lui un procédé d'estimation de mouvement d'un objet massique poly-articulé, composé d'une pluralité de segments reliés par au moins une articulation. Ce procédé comporte les étapes suivantes : acquisition de mesures inertielles issues d'au moins un module de capture fixe par rapport à un segment, lesdites mesures étant exprimées dans un repère de mesure fixe par rapport audit segment ; détermination à partir desdites mesures d'un vecteur effort extérieur à l'objet massique de manière à contrôler un modèle physique de cet objet, ledit modèle représentant l'objet massique ; estimation des grandeurs cinématiques associées aux segments de l'objet en appliquant le principe fondamental de la dynamique avec le vecteur d'effort déterminé à l'étape précédente.

**EXPOSÉ DE L'INVENTION**

L'invention a pour objectif de permettre d'obtenir des mouvements réalistes d'un mannequin numérique représentant une personne en utilisant un faible nombre de capteurs sur la personne, mais sans utiliser d'approche se reposant sur une base de données.

Elle propose pour ce faire un procédé de commande temps-réel d'une structure massique articulée à partir de données représentatives du mouvement d'un objet. Le procédé comprend les étapes d'acquisition de mesures réalisées à l'aide de capteurs de mouvement fixés sur l'objet, et de détermination de points cibles à partir des mesures acquises. Il comprend en outre les étapes :

- de détermination d'objectifs à remplir pour la commande de la structure massique articulée, les objectifs étant hiérarchisés et l'objectif de plus haut niveau étant l'atteinte des points cibles par la structure massique articulée ; et
- de détermination, par résolution en temps réel d'un problème d'optimisation, d'une commande à appliquer à la structure massique poly-articulée permettant de remplir les objectifs hiérarchisés.

Certains aspects préférés mais non limitatifs de ce procédé sont les suivants :

- la résolution du problème d'optimisation comprend la minimisation d'une erreur sur l'atteinte des points cibles par la structure poly-articulée ;
- les objectifs hiérarchisés comprennent un objectif de maintien de l'équilibre de la structure poly-articulée en tant qu'objectif de niveau inférieur à l'objectif de plus haut niveau ;
- les objectifs hiérarchisés comprennent un objectif de résolution de problèmes de redondance de la structure massique poly-articulée en tant qu'objectif de niveau inférieur à l'objectif de plus haut niveau ;
- il comprend en outre la détermination d'une ou plusieurs contraintes à respecter pour la commande de la structure massique poly-articulée, la détermination de la commande à appliquer à la structure massique poly-articulée étant réalisée par résolution d'un problème d'optimisation permettant de remplir les objectifs hiérarchisés tout en respectant la ou les contraintes ;
- le nombre de capteurs de mouvement fixés sur l'objet est compris entre trois et six ;
- la structure massique articulée est un modèle numérique de l'objet et il comprend en outre une représentation des mouvements de l'objet dans une simulation numérique au moyen de l'animation de la structure massique articulée dans la simulation numérique selon la commande déterminée.

L'invention s'étend à un produit programme d'ordinateur ainsi qu'à un système tous deux spécifiquement configurés pour permettre la mise en œuvre du procédé.

**BRÈVE DESCRIPTION DES DESSINS**

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :

- la figure 1 représente un exemple de structure massique poly-articulée utilisée comme modèle physique (au sens des lois de la physique) d'un corps humain ;
- la figure 2 est un schéma d'un système conforme à l'invention pour le suivi de mouvement d'un objet sur lequel des capteurs sont fixés ;
- la figure 3 est un schéma représentant les étapes du procédé selon l'invention, mises en œuvre à chaque pas de temps de calcul de la configuration à donner à la structure poly-articulée pour reproduire les mouvements de l'objet.

**EXPOSÉ DÉTAILLÉ DE MODES DE RÉALISATION PARTICULIERS**

L'invention concerne la commande d'une structure massique articulée à partir de données représentatives du mouvement d'un objet. Des capteurs de mouvement sont fixés sur l'objet et l'on souhaite asservir la structure massique articulée pour qu'elle reproduise les mouvements de l'objet.

L'objet est typiquement un corps humain ou le corps d'un animal, voire une seule partie d'un tel corps. L'invention n'est toutefois pas limitée à ces exemples, et s'étend ainsi au suivi du mouvement de tout objet mouvant pouvant être représenté par une structure articulée.

La structure massique articulée est composée de segments reliés par au moins une articulation. Elle est utilisée comme modèle physique de l'objet dont on suit le mouvement. Il s'agit typiquement d'une structure poly-articulée.

La structure massique articulée peut être un modèle numérique de l'objet. L'objectif peut alors être la représentation des mouvements de l'objet dans une simulation numérique, au moyen de l'animation de la structure massique articulée dans la simulation numérique selon la commande déterminée par le procédé selon l'invention.

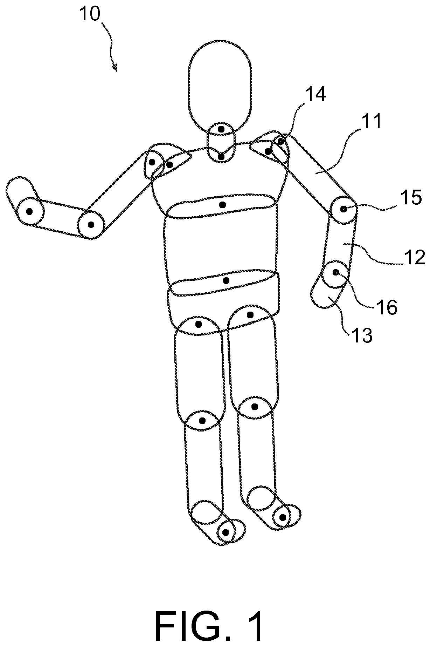

La figure 1 donne un exemple de structure massique poly-articulée 10 pouvant être utilisée comme modèle physique du corps humain dans une mise en œuvre de l'invention. La structure massique poly-articulée est composée de segments reliés par des articulations. Un segment désigne un objet rigide ou supposé rigide qui est défini par une géométrie (c'est-à-dire par une forme volumique bien définie), une masse et une inertie. Une articulation désigne une liaison entre deux segments. Cette liaison définit la configuration relative que peut avoir un segment par rapport à un segment auquel il est lié. Une articulation est définie par un ou plusieurs degrés de libertés représentatifs notamment des caractéristiques de centres, d'axes ou de butées.

Dans l'exemple de la figure 1, la structure poly-articulée 10 est composée de vingt-et-un segments reliés par vingt articulations. Ainsi, à titre d'exemple, la main est modélisée par un premier segment 13 relié par une articulation 16 à un deuxième segment 12 correspondant à l'avant-bras. L'articulation 16 possède par exemple trois degrés de liberté. Le segment 12 correspondant à l'avant-bras est relié par une articulation 15 à deux degrés de liberté au segment 11 correspondant au bras. Le segment 11 est quant à lui relié à la clavicule par une articulation 14 à trois degrés de libertés.

Cette structure 10 modélise un corps humain, par exemple de la manière suivante :

- Le bassin sert de référence. C'est le corps racine de la chaine poly-articulée.

- Le bassin présente 6 degrés de liberté (3 translations et 3 rotations) par rapport à l'environnement.
- Le tronc est composé de 3 segments rigides.
- Les doigts ne sont pas modélisés (ils sont rigidement fixés aux paumes).
- Les clavicules sont modélisées comme des segments rigides articulés au niveau du sternum.
- Le cou est un segment rigide
- La tête est un seul segment articulé sur le cou.
- Les pieds sont décomposés en deux parties : la plante et le talon d'un côté, les métatarses et les orteils de l'autre.
- La modélisation cinématique comprend 21 segments et 20 liaisons pour un total de 47 degrés de liberté internes.
- Chaque articulation est limitée par des butées articulaires.
- Les géométries des segments sont des segments dilatés ou des quadrilatères dilatés.
- Les inerties sont calculées à partir des géométries de chaque segment.

Outre la définition de la structure articulée (les géométries de chaque segment, les masses et les inerties de chaque segment et les paramètres des articulations : degrés de liberté, centre, axes, valeur des butées), les paramètres nécessaires à la mise en œuvre du procédé selon l'invention comprennent :

- La définition de la gravité (direction et norme du champ de pesanteur), dont l'effet (le poids) est représenté par des efforts extérieurs appliqués sur le centre de masse des segments de la structure articulée.
- La définition du sol. Cette définition de l'environnement comprend la géométrie du sol (reliefs, escaliers etc...), et un repère de référence attaché au sol. Elle permet notamment de détecter les collisions potentielles entre la structure articulée et l'environnement.
- La définition du comportement en frottement de la structure articulée sur l'environnement. Par exemple, si ce comportement est modélisé par une loi de Coulomb, le coefficient de frottement est donné.

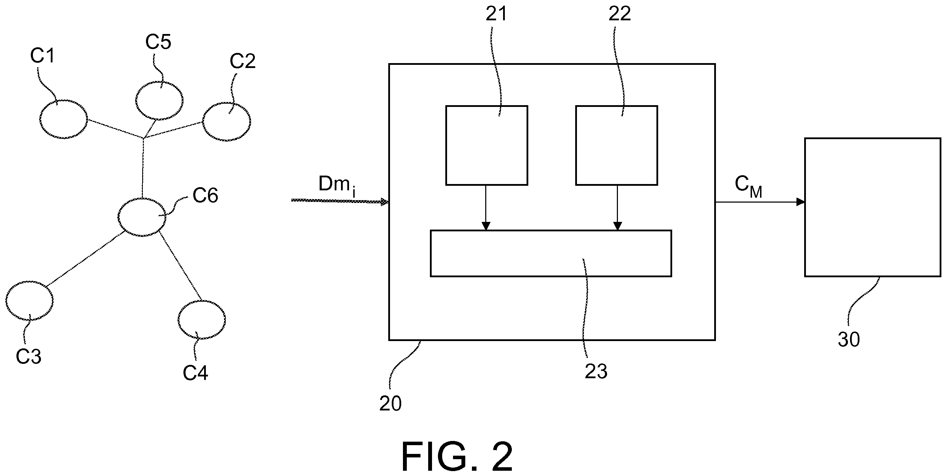

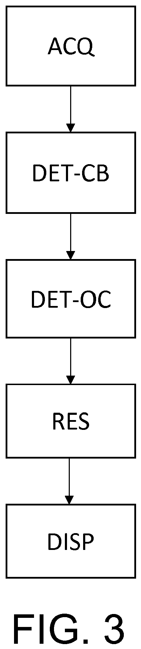

En référence maintenant aux figures 2 et 3, l'invention propose un procédé de commande d'une structure massique articulée à partir de données représentatives du mouvement d'un objet. Les étapes de ce procédé sont mises en œuvre à chaque pas de temps de calcul de la configuration à donner à la structure poly-articulée pour reproduire les mouvements de l'objet.

Le procédé comprend une première étape « ACQ » d'acquisition de mesures $Dm_i$ issues de capteurs de mouvement fixés sur l'objet.

Le nombre de capteurs de mouvement est réduit par rapport aux techniques d'animation conventionnelles. Prenant l'exemple du suivi de mouvement d'une personne, ce nombre est avantageusement compris entre trois et six. La personne peut notamment être équipée de cinq capteurs fixés rigidement sur son corps. En référence à la figure 2, on retrouve par exemple un capteur $C1$, $C2$ sur chaque main, un capteur $C3$, $C4$ sur chaque pied et un capteur $C5$ sur la tête. On peut également utiliser un sixième capteur $C6$ situé sur le bassin.

Les capteurs $C1$-$C6$ sont avantageusement des capteurs permettant de localiser de manière absolue dans l'espace, en position et en rotation, les différentes parties du corps. A titre d'exemple non limitatif, il s'agit de marqueurs réfléchissants, repérés en position et en orientation par un système de caméras infra-rouges. Les informations des caméras sont collectées par un PC et un logiciel permet d'estimer la position et l'orientation des capteurs en temps réel à partir des informations collectées. Les positions/orientations sont définies par rapport à un repère fixe déterminé au moment de la calibration des caméras.

En variante, les capteurs peuvent être des capteurs relatifs fournissant une localisation, en position et en rotation, dans un repère relatif à un élément central, ou encore une solution hybride entre capteurs absolus et capteurs relatifs.

Les mesures $Dm_i$ réalisées à l'aide des différents capteurs $C1$-$C6$ (des données de localisation, éventuellement associées à des degrés de confiance) sont acquises par une unité de traitement 20. Cette unité de traitement 20 est configurée pour, au cours d'une étape « DET-CB », déterminer des points cibles à partir des mesures acquises. Ces points cibles correspondent à la localisation, en position et en orientation, des différents capteurs $C1$-$C6$ fixés sur l'objet.

Puis, au cours d'une étape « DET-OC », l'unité de traitement 20 procède à une étape de détermination d'objectifs à remplir pour la commande de la structure massique poly-articulée. Plusieurs objectifs sont définis lors de cette étape, par exemple au sein d'un module de détermination d'objectifs 21 de l'unité de traitement 20.

Les objectifs sont hiérarchisés en fonction de leur degré de priorité, l'objectif de plus haut niveau étant l'atteinte des points cibles par la structure massique poly-articulée. En d'autres termes, l'objectif le plus prioritaire retenu dans l'invention consiste à ce que la structure articulée soit commandée selon une loi de commande lui permettant de se superposer aux points cibles issues des données des capteurs de mouvement. Ainsi, les segments de la structure articulée associés aux mains, aux pieds, à la tête et, le cas échéant, au bassin, doivent à l'issue de cette commande être localisés, en position et en orientation, conformément aux localisations des capteurs. Cet objectif étant le plus prioritaire, la latence ou l'erreur possible dans l'atteinte des points cibles sont ainsi réduites.

Conjointement à la détermination d'objectifs, l'unité de traitement peut également procéder lors de l'étape « DET-OC » à la détermination d'une ou plusieurs contraintes à respecter pour la commande de la structure massique poly-articulée. Ces contraintes sont par exemple définies au sein d'un module de détermination de contraintes 22 de l'unité de traitement 20.

Après formulation des objectifs hiérarchisés, et le cas échéant d'une ou plusieurs contraintes, l'unité de traitement 20, et plus particulièrement un module de résolution 23 de celle-ci, en liaison avec les modules de détermination d'objectifs et de contraintes 21, 22, vient procéder à une étape « RES » de détermination, par résolution d'un problème d'optimisation, notamment un problème d'optimisation quadratique, d'une commande à appliquer à la structure massique poly-articulée permettant de remplir les objectifs hiérarchisés, le cas échéant tout en respectant la ou les contraintes. Cette commande à appliquer s'exprime par exemple dans un espace dit articulaire comme un ensemble d'actionnement à appliquer à chacune des articulations de la structure. Cet ensemble d'actionnement entraîne un déplacement en rotation et en translation de chacun des segments par rapport au repère de référence, notamment lorsque les capteurs de mouvement permettent de localiser de manière absolue dans le repère de référence la partie du corps sur laquelle ils sont fixés.

L'objectif prioritaire étant l'atteinte des points cibles, la résolution du problème d'optimisation comprend la minimisation d'une erreur sur l'atteinte des points cibles par la structure poly-articulée. En d'autres termes, on cherche à minimiser l'erreur entre la localisation de chaque point cible et celle du segment correspondant de la structure articulée (mains, pieds, tête et, le cas échéant, bassin).

Cet objectif prioritaire d'atteinte de la position/orientation d'un capteur i par un membre x du mannequin peut s'exprimer sous la forme d'un asservissement en accélération :

$$\min_{\ddot{q}} \|\ddot{x} - \ddot{x}^{des}\|$$

- x(q) est la position/orientation cartésienne du membre,
- q correspond à l'état articulaire des liaisons de la structure articulée,
- $\ddot{q}$ sont les accélérations articulaires des liaisons,
- $\ddot{x}^{des}$ est l'accélération cartésienne désirée du membre et peut s'exprimer : ∘

$$\ddot{x}^{des} = K(x - ci) + D(\dot{x} - \dot{ci}),$$

∘ K et D sont des facteurs de raideur et d'amortissement (à fournir au problème),
∘ ci et $\dot{ci}$ sont la position et la vitesse du capteur i.

Dans un mode de réalisation de l'invention, les objectifs hiérarchisés comprennent un objectif de maintien de l'équilibre de la structure poly-articulée en tant qu'objectif de niveau inférieur à l'objectif de plus haut niveau.

Dans un tel cas de figure, la résolution du problème d'optimisation peut alors comprendre la minimisation d'une erreur sur le respect d'une consigne de position du centre de masse de la structure poly-articulée. Une telle consigne permet de déterminer les actionnements adéquats permettant de positionner correctement le corps racine (et les éléments proches de la racine) de la structure poly-articulée.

Cet objectif du maintien du centre de masse $x_{com}$ peut s'exprimer sous la forme $\min_{\ddot{q}} \|\ddot{x}_{com} - \ddot{x}_{com}^{des}\|$, avec :

- $\ddot{x}_{com}$, l'accélération courante du centre de masse,
- $\ddot{x}_{com}^{des}$, la consigne en accélération (à fournir au problème).
- La consigne en accélération peut aussi se formuler $\ddot{x}_{com}^{des} = K_{com}(x_{com} - x_{com}^{des}) + D_{com}(\dot{x}_{com} - \dot{x}_{com}^{des})$ où $K_{com}$ et $D_{com}$ sont des facteurs de raideur et d'amortissement et $x_{com}^{des}$ et $\dot{x}_{com}^{des}$ sont des consignes en position et vitesse du centre de masse.
- Les facteurs et consignes sont déterminés selon des heuristiques et fournis au problème par un expert du domaine en fonction du type d'application.

Dans un mode de réalisation, complémentaire ou non du mode de réalisation précédent, les objectifs hiérarchisés comprennent un objectif de résolution de problèmes de redondance de la structure massique poly-articulée en tant qu'objectif de niveau inférieur à l'objectif de plus haut niveau. Les problèmes de redondance apparaissent lorsque la dimension de l'espace cartésien des consignes (fournies par les capteurs) est plus faible que la dimension de l'espace articulaire de la structure poly-articulée.

Dans un tel cas de figure, la résolution du problème d'optimisation peut comprendre la minimisation d'une erreur sur le respect d'une posture type de la structure poly-articulée. On vient ainsi privilégier une posture type par rapport à d'autres postures, qui certes remplissent l'objectif de plus haut niveau, mais correspondent à des mouvements non réalistes. La posture type peut être choisie en fonction du type d'application (par exemple selon que la personne marche ou soit en train de skier). A titre d'exemple, le segment main de la structure articulée étant correctement asservi en localisation, différentes solutions redondantes d'articulations du coude sont possibles. La prise en compte de l'objectif de résolution des problèmes de redondance permet de retenir une solution parmi les solutions redondantes, typiquement la solution la plus réaliste approchant la posture type.

L'objectif du maintien de la posture type peut s'exprimer sous la forme $\min_{\ddot{q}} \|\ddot{q} - \ddot{q}^{des}\|$, avec :

- $\ddot{q}^{des}$ l'accélération articulaire désirée des liaisons, qui peut s'exprimer selon :

  ○

$$\ddot{q}^{des} = P(q - q^{des}) + B(\dot{q} - \dot{q}^{des}),$$

  ○ P et B sont des facteurs de raideur et d'amortissement (à fournir au problème),
  ○ $q^{des}$ et $\dot{q}^{des}$ sont des postures et vitesses de postures désirées (à choisir en fonction du type d'application et à fournir au problème).

La résolution du problème d'optimisation peut mettre en œuvre une combinaison des objectifs hiérarchisés sous la forme d'une somme pondérée où le poids associé à un objectif est fonction de son niveau hiérarchique. Le poids le plus important est alors attribué au niveau prioritaire d'atteinte des points cibles.

Alternativement, la résolution du problème d'optimisation peut mettre en œuvre, pour chaque objectif de niveau inférieur au plus haut niveau, une projection dans l'espace nul de chaque objectif de niveau supérieur. Cette projection permet à une commande permettant de remplir l'objectif de niveau inférieur à l'objectif de plus haut niveau de ne pas interférer avec une ou des commandes permettant de remplir des objectifs de niveau supérieur. Reprenant l'exemple de la localisation de la main et des différentes solutions d'articulation du coude, cette projection assure que les différentes solutions d'articulation du coude assurent toujours le respect de la bonne localisation de la main (objectif prioritaire) et le maintien de l'équilibre (dans le cas où cet objectif est plus prioritaire que celui de la gestion des redondances).

Une telle technique de projection est par exemple utilisée dans le domaine de la robotique. On pourra à cet égard se référer à l'article de A. Dietrich et al. intitulé « An Overview of Null Space Projections for Redundant, Torque Controlled Robots », The International Journal of Robotics Research, 34(11):1385-1400, Septembre 2015.

Outre les trois objectifs présentés ci-dessus, la formulation du problème d'optimisation peut, à titre d'exemple, comprendre les quatre contraintes suivantes : le respect des équations dynamiques du mouvement, le non-glissement des contacts, l'accélération nulle des contacts et le respect des limites articulaires.

La contrainte portant sur le respect des équations dynamiques du mouvement peut s'écrire sous la forme :

$$M(q) + C(q, \dot{q})\dot{q} + g(q) = \tau + \tau_{ext}$$

- q correspond à l'état articulaire des liaisons de la structure articulée,
- $\tau$ correspond aux couples articulaires de la structure articulée,
- $\tau_{ext}$ correspond aux efforts extérieurs,
- M correspond à la matrice d'inertie de la structure articulée,
- C correspond à la matrice de Coriolis de la structure articulée,
- g représente les efforts dus à la gravité.

La contrainte de non-glissement des contacts peut s'écrire sous la forme :

$$f_t \leq \mu \, f_n$$

- $f_t$ est la force tangentielle au point de contact,
- $f_n$ est la force normale au point de contact,
- $\mu$ est le coefficient de frottement,
- Il est nécessaire de linéariser cette contrainte en discrétisant un cône de friction.

La contrainte d'accélération nulle des contacts peut s'écrire sous la forme $J\ddot{q} + \dot{j}\dot{q} = 0$ où J est la jacobienne des points de contact.

La contrainte de respect des limites articulaires peut quant à elle s'exprimer sous la forme $q^{min} \leq q \leq q^{max}$.

Ainsi, pour un pas de temps donné, le problème d'optimisation lié au comportement de la structure articulée est formulé et résolu. Comme décrit ci-dessus, les objectifs du problème d'optimisation peuvent être :

- L'atteinte des points cibles dans l'environnement, dont la position et l'orientation sont fournies par les capteurs de mouvements,
- Le maintien de l'équilibre, et
- La gestion de la redondance.

Les contraintes du problème d'optimisation s'expriment sous la forme d'efforts : des efforts extérieurs, les efforts de gravité et les efforts de contact, et des efforts internes liés aux articulations de la structure articulée.

En résolvant le problème d'optimisation en temps réel, l'unité de traitement 20 détermine ainsi la commande $C_M$ à appliquer à la structure articulée. Ce problème peut notamment être résolu avec un solveur de programmation quadratique QP (pour « Quadratic Programming »).

La commande $C_M$ déterminée par l'unité de traitement 20 peut être utilisée afin de réaliser une analyse du mouvement. En effet, à partir de la connaissance du mouvement, des informations spécifiques peuvent être extraites pour des besoins applicatifs particuliers. Cette commande $C_M$ peut également être utilisée pour réaliser une reconstruction complète du mouvement en vue de l'afficher sur un écran 30, par exemple celui d'un ordinateur ou d'un casque de réalité virtuelle. Le mouvement peut être modifié ou amplifié pour obtenir des effets désirés. Le procédé comprend alors une étape « DISP » d'animation, selon la commande déterminée, de la structure massique poly-articulée dans une simulation numérique 3D.

Le procédé selon l'invention est ainsi capable, pour réaliser la commande de la structure articulée, de prendre en compte les données provenant de capteurs de mouvement. Il est en outre capable, dans le cadre d'une simulation physique d'une structure articulée, de prendre en compte l'effet de la gravité, de détecter les collisions entre les géométries des segments de la structure articulée et la géométrie de l'environnement et de résoudre les contacts en générant des efforts s'opposant aux pénétrations entre les segments de la structure articulée et l'environnement.

A cet égard, les collisions entre les géométries des segments de la structure articulée et la géométrie de l'environnement sont détectées à chaque pas de temps pour une configuration de la structure donnée. Cette détection peut être basée sur la détection de pénétration des géométries comme sur la détection de proximité selon un seuil donné. Les informations de collisions obtenues sont le point et la normale de chaque contact. Les informations de collision sont utilisées pour générer des efforts conduisant à empêcher les pénétrations entre les géométries des segments et celle de l'environnement. Une modélisation du frottement (par exemple par une loi de Coulomb) permet, de plus, de simuler l'adhérence du segment sur l'environnement et contraindre le glissement. Les efforts résultants sont appelés efforts de contact.

Le procédé selon l'invention peut ainsi être mis en œuvre avec le concours d'un moteur de simulation physique temps-réel, tel que le moteur XDE (pour « eXtended Dynamic Engine ») de la Demanderesse, qui permet de réaliser des simulations dans un environnement virtuel en venant affecter des contraintes physiques à une structure articulée, détecter les collisions et gérer les contacts. De la sorte, la structure articulée peut suivre un faible nombre de capteurs dans l'espace tout en gérant les problèmes d'équilibre et de redondance.

Ce procédé se place en rupture par rapport aux procédés existants du domaine de l'animation car il ne se base pas sur des techniques courantes de cinématique inverse. Ce procédé ne se base pas non plus sur une approche « data-driven ». Au contraire, ce procédé se base sur des techniques de commande hiérarchique issues du domaine de la robotique (voir notamment l'article précité de A. Dietrich et al.) et dans lesquelles les données d'entrée sont ici fournies par des capteurs de mouvement. Utilisées conformément à l'invention, ces techniques permettent de rajouter de l'intelligence au contrôle de la structure articulée réalisant le suivi de mouvement, en résolvant notamment les problèmes de gestion d'équilibre et de redondance ce qui autorise ainsi l'utilisation d'un faible nombre de capteurs de mouvement.

L'invention n'est pas limitée au procédé précédemment décrit, mais s'étend également à un système de commande d'une structure massique poly-articulée à partir de données représentatives du mouvement d'un objet, ledit système étant configuré pour permettre la mise en œuvre du procédé. En référence à la figure 2, il comprend ainsi une unité de traitement 20 configurée pour acquérir des mesures issues de capteurs de mouvement C1-C6 fixés sur l'objet et pour déterminer des points cibles à partir des mesures acquises. Cette unité de traitement est configurée pour déterminer, par résolution d'un problème d'optimisation, une commande $C_M$ à appliquer à la structure massique poly-articulée, ladite commande permettant de remplir des objectifs hiérarchisés, où l'objectif de plus haut niveau est l'atteinte des points cibles par la structure massique poly-articulée.

L'invention concerne également un produit programme d'ordinateur comportant des instructions de code de programme pour l'exécution du procédé selon l'invention lorsque ledit programme est exécuté sur un ordinateur.

Cette invention peut être utilisée notamment dans le cadre de la rééducation fonctionnelle, de l'actimétrie, de la formation sportive, des jeux vidéo, du diagnostic thérapeutique, du suivi thérapeutique, de la formation professionnelle au geste technique, de l'ergonomie des produits, de l'ergonomie du poste de travail, de l'animation, du suivi de troupes militaires et/ou du suivi d'acteurs de la sécurité civile.

## Revendications

1. Procédé de commande temps-réel d'une structure massique articulée (10) pour qu'elle reproduise un mouvement d'un objet, le procédé comprenant les étapes :

   - d'acquisition (ACQ) de mesures réalisées à l'aide de capteurs de mouvement (C1-C6) fixés sur l'objet ; et
   - de détermination (DET-CB) de points cibles à partir des mesures acquises ;

   le procédé étant **caractérisé en ce qu'**il comprend en outre les étapes :

   - de détermination (DET-OC) d'objectifs à remplir pour la commande de la structure massique articulée, lesdits objectifs ayant chacun un degré de priorité et étant hiérarchisés en fonction de leur degré de priorité, lesdits objectifs comprenant un objectif de plus haut niveau et au moins un objectif de niveau inférieur à l'objectif de plus haut niveau, l'objectif de plus haut niveau étant l'atteinte des points cibles par la structure massique articulée ; et

- de détermination (RES), par résolution en temps réel d'un problème d'optimisation, d'une commande ($C_M$) à appliquer à la structure massique poly-articulée permettant de remplir lesdits objectifs.

2. Procédé selon la revendication 1, dans lequel la résolution du problème d'optimisation comprend la minimisation d'une erreur sur l'atteinte des points cibles par la structure poly-articulée.

3. Procédé selon l'une des revendications 1 et 2, dans lequel l'au moins un objectif de niveau inférieur à l'objectif de plus haut niveau comprend un objectif de maintien de l'équilibre de la structure poly-articulée.

4. Procédé selon la revendication 3, dans lequel la résolution du problème d'optimisation comprend la minimisation d'une erreur sur le respect d'une consigne de position du centre de masse de la structure poly-articulée.

5. Procédé selon l'une des revendications 1 à 4, dans lequel l'au moins un objectif de niveau inférieur à l'objectif de plus haut niveau comprend un objectif de résolution de problèmes de redondance de la structure massique poly-articulée.

6. Procédé selon la revendication 5, dans lequel la résolution du problème d'optimisation comprend la minimisation d'une erreur sur le respect d'une posture type de la structure poly-articulée.

7. Procédé selon l'une des revendications 1 à 6, comprenant en outre la détermination d'une ou plusieurs contraintes à respecter pour la commande de la structure massique poly-articulée et dans lequel la détermination de la commande à appliquer à la structure massique poly-articulée est réalisée par résolution d'un problème d'optimisation permettant de remplir lesdits objectifs tout en respectant la ou les contraintes.

8. Procédé selon l'une des revendications 1 à 7, dans lequel la résolution du problème d'optimisation met en œuvre une combinaison desdits objectifs sous la forme d'une somme pondérée où le poids associé à un objectif est fonction de son niveau de priorité.

9. Procédé selon l'une des revendications 1 à 7, dans lequel la résolution du problème d'optimisation met en œuvre, pour chaque objectif de niveau inférieur à l'objectif de plus haut niveau, une projection dans l'espace nul de chaque objectif de niveau supérieur, permettant à une commande permettant de remplir l'objectif de niveau inférieur à l'objectif de plus haut niveau de ne pas interférer avec une ou des commandes permettant de remplir un ou des objectifs de niveau supérieur.

10. Procédé selon l'une des revendications 1 à 9, dans lequel la structure massique articulée est composée des segments reliés par des articulations et dans lequel la commande à appliquer comprend un actionnement de chacune des articulations, entraînant un déplacement en rotation et en translation de chacun des segments par rapport à un repère de référence.

11. Procédé selon l'une des revendications 1 à 10, dans lequel le nombre de capteurs de mouvement fixés sur l'objet est compris entre trois et six.

12. Procédé selon l'une des revendications 1 à 11, dans lequel la structure massique articulée est un modèle numérique de l'objet et comprenant en outre une représentation (DISP) des mouvements de l'objet dans une simulation numérique au moyen de l'animation de la structure massique articulée dans la simulation numérique selon la commande déterminée.

13. Système de commande temps-réel d'une structure massique articulée pour qu'elle reproduise un mouvement d'un objet, comportant une unité de traitement (20) configurée pour acquérir des mesures issues de capteurs de mouvement (C1-C6) fixés sur l'objet et pour déterminer des points cibles à partir des mesures acquises, **caractérisé en ce que** l'unité de traitement est en outre configurée pour déterminer, par résolution temps-réel d'un problème d'optimisation, une commande ($C_M$) à appliquer à la structure massique articulée permettant de remplir des objectifs ayant chacun un degré de priorité et étant hiérarchisés en fonction de leur degré de priorité, lesdits objectifs comprenant un objectif de plus haut niveau et au moins un objectif de niveau inférieur à l'objectif de plus haut niveau, l'objectif de plus haut niveau étant l'atteinte des points cibles par la structure massique articulée.

14. Produit programme d'ordinateur comportant des instructions de code de programme pour l'exécution du procédé selon l'une des revendications 1 à 12 lorsque ledit programme est exécuté sur un ordinateur.

**Patentansprüche**

1. Verfahren zur Echtzeitsteuerung einer gelenkigen Massenstruktur (10), damit diese eine Bewegung eines Objekts reproduziert, wobei das Verfahren die folgenden Schritte umfasst:

- Erfassung (ACQ) von Messungen, die mit Hilfe von an dem Objekt befestigten Bewegungssensoren (C1-C6) durchgeführt werden, und
- Bestimmung (DET-CB) von Zielpunkten aus den erfassten Messungen;

wobei das Verfahren **dadurch gekennzeichnet ist, dass** es ferner die folgenden Schritte umfasst:

- Bestimmung (DET-OC) von Zielen, die für die Steuerung der gelenkigen Massenstruktur zu erfüllen sind, wobei die Ziele jeweils einen Prioritätsgrad aufweisen und entsprechend ihrem Prioritätsgrad hierarchisiert werden, wobei die Ziele ein Ziel der höchsten Ebene und wenigstens ein Ziel einer niedrigeren Ebene als das Ziel der höchsten Ebene umfassen, wobei das Ziel der höchsten Ebene das Erreichen der Zielpunkte durch die gelenkige Massenstruktur ist; und
- Bestimmung (RES), durch Echtzeitlösung eines Optimierungsproblems, einer Steuerung ($C_M$), die auf die mehrgelenkige Massenstruktur angewendet werden soll, um die Ziele zu erfüllen.

2. Verfahren nach Anspruch 1, wobei die Lösung des Optimierungsproblems die Minimierung eines Fehlers beim Erreichen der Zielpunkte durch die mehrgelenkige Massenstruktur umfasst.

3. Verfahren nach einem der Ansprüche 1 und 2, wobei das wenigstens eine Ziel der niedrigeren Ebene als das Ziel der höchsten Ebene ein Ziel zur Aufrechterhaltung des Gleichgewichts der mehrgelenkigen Struktur umfasst.

4. Verfahren nach Anspruch 3, wobei die Lösung des Optimierungsproblems die Minimierung eines Fehlers bei der Einhaltung einer Vorgabe für die Position des Massenmittelpunkts der mehrgelenkigen Struktur umfasst.

5. Verfahren nach einem der Ansprüche 1 bis 4, wobei das wenigstens eine Ziel der niedrigeren Ebene als das Ziel der höchsten Ebene ein Ziel zur Lösung von Redundanzproblemen der mehrgelenkigen Massenstruktur umfasst.

6. Verfahren nach Anspruch 5, wobei die Lösung des Optimierungsproblems die Minimierung eines Fehlers bei der Einhaltung einer typischen Haltung der mehrgelenkigen Struktur umfasst.

7. Verfahren nach einem der Ansprüche 1 bis 6, ferner umfassend die Bestimmung einer oder mehrerer Einschränkungen, die bei der Steuerung der mehrgelenkigen Massenstruktur einzuhalten sind, und wobei die Bestimmung der auf die mehrgelenkige Massenstruktur anzuwendenden Steuerung durch die Lösung eines Optimierungsproblems erfolgt, das die Erfüllung der Ziele unter Einhaltung der Einschränkung(en) ermöglicht.

8. Verfahren nach einem der Ansprüche 1 bis 7, wobei die Lösung des Optimierungsproblems eine Kombination der Ziele in Form einer gewichteten Summe einsetzt, wobei das Gewicht, das einem Ziel zugeordnet ist, von seiner Prioritätsstufe abhängt.

9. Verfahren nach einem der Ansprüche 1 bis 7, wobei die Lösung des Optimierungsproblems für jedes Ziel der niedrigeren Ebene als das Ziel der höchsten Ebene eine Nullraumprojektion jedes Ziels einer höheren Ebene umfasst, die es einem Befehl zur Erfüllung des Ziels einer niedrigeren Ebene als das Ziel der höchsten Ebene ermöglicht, nicht mit einem Befehl oder Befehlen zur Erfüllung eines Ziels oder von Zielen einer höheren Ebene zu interferieren.

10. Verfahren nach einem der Ansprüche 1 bis 9, wobei die gelenkige Massenstruktur aus Segmenten besteht, die durch Gelenke verbunden sind, und wobei der anzuwendende Befehl eine Betätigung jedes der Gelenke umfasst, die eine Dreh- und Translationsbewegung jedes der Segmente in Bezug auf eine Referenzmarkierung bewirkt.

11. Verfahren nach einem der Ansprüche 1 bis 10, wobei die Anzahl der am Objekt befestigten Bewegungssensoren zwischen drei und sechs liegt.

12. Verfahren nach einem der Ansprüche 1 bis 11, wobei die gelenkige Massenstruktur ein numerisches Modell des Objekts ist und ferner eine Darstellung (DISP) der Bewegungen des Objekts in einer numerischen Simulation mittels Animation der gelenkigen Massenstruktur in der numerischen Simulation gemäß dem festgelegten Befehl umfasst.

13. System zur Echtzeitsteuerung einer gelenkigen Massenstruktur, damit diese eine Bewegung eines Objekts reproduziert, umfassend eine Verarbeitungseinheit (20), die dazu ausgebildet ist, Messungen von Bewegungssensoren (C1-C6), die am Objekt befestigt sind, zu erfassen und Zielpunkte aus den erfassten Messungen zu bestimmen, **dadurch gekennzeichnet, dass** die Verarbeitungseinheit ferner dazu ausgebildet ist, durch Echtzeitlösung eines Optimierungsproblems einen Befehl ($C_M$) zu bestimmen, der auf die gelenkige Massenstruktur anzuwenden ist und es ermöglicht, Ziele zu erfüllen, die jeweils einen Prioritätsgrad haben und in Abhängigkeit von ihrem Prioritätsgrad hierarchisiert sind, wobei die Ziele ein Ziel der höchsten Ebene und wenigstens ein Ziel einer niedrigeren Ebene als das Ziel der höchsten Ebene umfassen, wobei das Ziel der höchsten Ebene das Erreichen der Zielpunkte durch die gelenkige Massenstruktur ist.

14. Computerprogrammprodukt, umfassend Programmcodeanweisungen zum Ausführen des Verfahrens nach einem der Ansprüche 1 bis 12, wenn das Programm auf einem Computer ausgeführt wird.

**Claims**

1. Method for real-time control of an articulated mass structure (10) so that it reproduces a movement of an object, the method comprising the steps of:

    - acquiring (ACQ) measurements made using motion sensors (C1-C6) attached to the object; and
    - determining (DET-CAB) target points from the measurements acquired;

    the method being **characterised in that** it further comprises the steps of:

    - determining (DET-OC) objectives to fulfil for the control of the articulated mass structure, said objectives each having a degree of priority and being arranged in a hierarchy according to the degree of priority thereof, said objectives comprising a highest level objective and at least one objective of a lower level than the highest level objective, the highest level objective being the attainment of the target points by the articulated mass structure; and
    - determining (RES), by real-time resolution of an optimisation problem, a command ($C_M$) to be applied to the poly-articulated mass structure to fulfil said objectives.

2. Method according to claim 1, wherein the resolution of the optimisation problem comprises minimising an error on the attainment of the target points by the poly-articulated structure.

3. Method according to one of claims 1 and 2, wherein the at least one objective of lower level than the highest level objective comprises an objective of maintaining the equilibrium of the poly-articulated structure.

4. Method according to claim 3, wherein the resolution of the optimisation problem comprises minimising an error on compliance with a position set-point of the centre of mass of the poly-articulated structure.

5. Method according to one of claims 1 to 4, wherein the at least one objective of lower level than the highest level objective comprising an objective of resolving redundancy problems of the poly-articulated mass structure.

6. Method according to claim 5, wherein the resolution of the optimisation problem comprises minimising an error on compliance with a typical posture of the poly-articulated structure.

7. Method according to one of claims 1 to 6, further comprising determining one or more constraints to be observed for the control of the poly-articulated mass structure and wherein the command to be applied to the poly-articulated mass structure is determined by resolving an optimisation problem making it possible to fulfil said objectives while complying with the constraint(s).

8. Method according to one of claims 1 to 7, wherein the resolution of the optimisation problem uses a combination of said objectives in the form of a weighted sum where the weight associated with an objective is dependent on the priority level thereof.

9. Method according to one of claims 1 to 7, wherein the resolution of the optimisation problem uses, for each objective of lower level than the highest level objective, a zero-space projection of each objective of higher level, enabling a command to fulfil the objective of lower level than the highest level objective not to interfere with one or more commands making it possible to fulfil one or more objectives of higher level.

10. Method according to one of claims 1 to 9, wherein the articulated mass structure is composed of segments connected by articulations and wherein the command to be applied comprises an actuation of each of the articulations, inducing a rotation and translation movement of each of the segments with respect to a reference point.

11. Method according to one of claims 1 to 10, wherein the number of motion sensors attached to the object is between three and six.

12. Method according to one of claims 1 to 11, wherein the articulated mass structure is a digital model of the object and further comprising a representation (DISP) of the movements of the object in a digital simulation by animating the articulated mass structure in the digital simulation according to the defined command.

13. System for real-time control of an articulated mass structure so that it reproduces a movement of an object, including a processing unit (20) configured to acquire measurements obtained from motion sensors (C1-C6) attached to the object and to determine target points from the measurements acquired, **characterised in that** the processing unit is furthermore configured to determine, by real-time resolution of an optimisation problem, a command ($C_M$) to be applied to the poly-articulated mass structure making it possible to fulfil said objectives each having a degree of priority and being arranged in a hierarchy according to the degree of priority thereof, said objectives comprising a highest level objective and at least one objective of a lower level than the highest level objective, the highest level objective being the attainment of the target points by the articulated mass structure.

14. Computer program product including program code instructions for executing the method according to one of claims 1 to 12 wherein said program is executed on a computer.

FIG. 1

FIG. 2

ACQ

↓

DET-CB

↓

DET-OC

↓

RES

↓

DISP

# FIG. 3

## RÉFÉRENCES CITÉES DANS LA DESCRIPTION

**Documents brevets cités dans la description**

- US 20150192413 A1
- WO 2013131990 A1
- FR 3000376 A1

**Littérature non-brevet citée dans la description**

- **A. DIETRICH et al.** An Overview of Null Space Projections for Redundant, Torque Controlled Robots. *The International Journal of Robotics Research,* Septembre 2015, vol. 34 (11), 1385-1400